# EUROPEAN PATENT APPLICATION

(11) **EP 4 094 695 A1**
(43) Date of publication of application: **30.11.2022**
(21) Application number: 21290034.4
(22) Date of filing: 28.05.2021
(51) Int. Cl.: A61B 8/08, A61B 8/00

(54) **ULTRASOUND IMAGING SYSTEM**

(71) Applicant: Koninklijke Philips N.V., 5656 AG Eindhoven (NL)
(72) Inventor: De Craene, Mathieu, 5656 AE Eindhoven (NL); Waechter-Stehle, Irina, 5656 AE Eindhoven (NL); Allain, Pascal, 5656 AE Eindhoven (NL); Holland Settlemier, Scott, 5656 AE Eindhoven (NL); Prater, David, 5656 AE Eindhoven (NL)
(74) Representative: Philips Intellectual Property & Standards

(57) **Abstract**

An ultrasound imaging system performs real-time image analysis of the 3D ultrasound images to identity anatomical structure and landmarks in the 3D images, so that it can be determined if a region of interest of a target anatomical structure is present. If the region of interest is not present, the location of the region of interest outside the field of view volume is inferred from the anatomical structure and landmarks that are detected within the field of view. A relative displacement between the reference anatomical plane and a position and orientation of the 3D ultrasound imaging probe can then be derived so that ultrasound guidance information can be given.

## Description

### FIELD OF THE INVENTION

This invention relates to ultrasound imaging systems, using an ultrasound probe.

### BACKGROUND OF THE INVENTION

The acquisition of ultrasound images, such as echocardiographic views, can be challenging for novice sonographers.

Correctly handling the probe and providing correct patient positioning, also accounting for the patient's breathing, is a skill that takes time to master. When acquiring apical views in echocardiography, a common pitfall is to place the probe too far from the Left Ventricle (LV) apex. This results in a plane that does not intersect the heart correctly, and the shape or the size of the LV can look altered.

Quantifying features from such an incorrect plane (e.g. computing the volume) will result in incorrect findings. In general, quantifying the heart shape, motion, or flow from an incorrect plane affects the reproducibility of ultrasound-based measurements. Thus, the imaging of incorrect planes may lead to reproducibility issues in the view itself, but also in any derived measurements when quantifying volumes, motion and flow.

There are many examples of guidance tools to assist ultrasound imaging, for example based on analysis of 2D ultrasound images, or using anatomical intelligence-based analysis of an ultrasound image feed. Such tools can provide simple indications regarding corrective shifts or tilting of the ultrasound probe.

However, there are issues with the accuracy of such a 2D anatomical intelligence-based solution to infer 3D geometry. Furthermore, such systems can only give guidance in respect of small changes in position or orientation of the ultrasound probe which are apparent from fields of view of the acquired images.

### SUMMARY OF THE INVENTION

The invention is defined by the claims.

According to examples in accordance with an aspect of the invention, there is provided an ultrasound imaging system, comprising:
a 3D ultrasound imaging probe for acquiring real-time 3D images of a field of view volume with the 3D ultrasound imaging probe manually positioned by a user;
a controller configured to:
   perform a real-time image analysis of the 3D images thereby to:
   identify anatomical structure and landmarks in the 3D images;
   based on the identified anatomical structure and landmarks, determine if a region of interest of a target anatomical structure is present;
   if, based on the identified anatomical structure and landmarks, it is determined that the region of interest is not present, to infer the location of the region of interest outside the field of view volume from the anatomical structure and landmarks that are detected within the field of view;
   identify the position of at least one reference anatomical plane for the target anatomical structure;
   determine a relative position between the reference anatomical plane and a position and orientation of the 3D ultrasound imaging probe; and
provide ultrasound guidance information based on the determined relative position.

The region of interest is for example a particular part of a target anatomical structure such as an organ or vessel, or a particular imaging plane. For example, a region of interest may be a heart valve or the 4-chambers plane, and the target anatomical structure is the heart. The region of interest is the part of the anatomy which needs to be imaged in order to enable a desired diagnostic evaluation. The region of interest may in some cases be the whole of an organ or it may only be a part of an organ or other structure.

The invention makes use of 3D anatomical intelligence to detect the anatomy of a target of interest (e.g. an organ, part of an organ, a valve) and the relative position/orientation between a reference anatomical plane (which is a desired plane to be imaged) of the target of interest (organ) and the ultrasound probe. Anatomical intelligence involves the use of 3D anatomical models which provide organ modeling, image slicing and quantification to make ultrasound exams easier to perform and more reproducible.

Using the obtained relative position (i.e. a relative translational and angular alignment), misalignment may be detected between the current acquisition position of the probe and the position required to capture a reference anatomical plane. This reference anatomical plane is for example a standard view used for diagnostic purposes.

An image of the region of interest, in particular a desired view of the region of interest, may not be reachable from the current imaging probe position. For example, in the case of the heart, the 3D anatomical intelligence can find the left ventricle (LV) long axis and predict the apex, to reveal that the target long axis and apex are outside of the current field of view. In this case, the region of interest is the apex and long axis of the left ventricle. Thus, the probe needs to be moved to capture the desired view.

The guidance may then comprise manual guidance advice to assist in capturing one of the reference anatomical planes i.e. reference views.

Thus, the invention in this example provides an approach by which anatomical intelligence can capture 3D positions of anatomical structure (e.g. based on landmarks) and anatomical planes, as well providing indications about how to optimally place a probe to the user. In other examples, the guidance may provide an indication of which reference images are achievable from the current probe location or which are the nearest achievable reference images.

The image analysis can run in the background. In this way, anatomical intelligence is used as part of a continuous background task, rather than being performed only on an isolated set of volumes. This can make echocardiography more intuitive by understanding what the sonographer is trying to achieve, giving hints about how to optimize the probe placement, and proposing tuned acquisition modes for standard parts of the echocardiographic exam.

A number of workflow improvements can also be derived that benefit from the 3D anatomical knowledge. Workflow improvements may be defined interactively. Shortcuts may for example be provided to one or several (consecutive) reference views, that are identified as feasible from the current probe position, and for which the plane or the focus area have been computed by anatomical intelligence. These shortcuts may be displayed to the user.

Preset acquisitions may for example be proposed centered on anatomical landmarks, such as the valves.

The controller is for example further configured to perform an image quality assessment for the field of view volume and discard images determined as unsuitable for the identification of anatomical structure.

The image quality assessment may for example involve using a trained classifier that recognizes correct fitting of the model anatomy onto the image and gives it a score. An alternative is a consistency measure that ensures stability of the anatomy detection to remove outliers.

This provides a quality pre-processing step that ensures the current volume is of sufficient quality e.g. for locating key landmarks and anatomical structures. This avoids noisy predictions that would randomly detect points incorrectly for example at opposite corners of the volume, which would give a disturbing guidance experience for the user.

The controller may be configured to determine the relative position by:
identifying one or more main directions of the anatomy of the target of interest; and
determining a distance and orientation between the one or more main directions and one or more axes of the ultrasound imaging probe.

The relative position may thus include both a distance to the reference anatomical plane as well as an orientation. Thus, the guidance may include translation instructions and tilting instructions.

The aim is to assist imaging through a reference point (such as the apex of the heart) and a with a particular orientation (such as along the long axis of the heart and pointing to the mitral valve). If an image is known to include the apex and is aligned with the long axis, reliable measurements may be taken from the image such as chamber volumes or structure areas.

The user will initially be pointing the probe towards another origin and in another direction. Computing the displacement between both the desired and the current direction involves computing the distance between the heart apex and the cone apex for the current field of view, and computing an angular distance between the two directions (i.e. between two vectors).

The guidance information may provide:
advice as to whether or not imaging a desired reference anatomical plane of the region of interest is achievable from the current position and orientation of the ultrasound imaging probe; or
recommendations about the nearest achievable acquisitions of a reference anatomical plane of the region of interest from the current position and orientation of the ultrasound probe; or
warnings or guidance to indicate required correction of the probe position to enable acquisition of a desired reference anatomical plane.

By these measures, the correctness of the planes can be improved and differences between skilled sonographers and novices can be reduced. This is currently achieved by face to face training, which scales difficultly with the rapidly growing demand for (cardiac) echo exams. The system of the invention assists a novice user to take advantage of the 3D localization capabilities of the system to correct his/her probe positioning skills, before moving back to more portable system that is not provided with the guidance capability.

The warnings or guidance enable a novice sonographer to use such displayed warnings to correct the probe position. This can help novice users who are being trained on premium systems to tune their acquisition skills, which will be helpful on all devices.

The controller may be configured, in response to an input command, to acquire simultaneously a set of views of reference anatomical planes.

For example, an input function (e.g. a dedicated button) may be provided for acquiring at once all reference views from the apical window (2, 3 and 4 Chambers). The system may also allow a user to zoom around a feature of interest such as a valve to visualize it in 3D, using either B-Mode or color imaging. In respect of a valve, the gate position and Doppler lines can be placed automatically, taking into account 3D anatomical knowledge of the valve location.

The controller may be configured to detect if the region of interest is present by using a landmarks detection algorithm for tracking key anatomical points. The key anatomical points for example comprise the heart valves for cardiac imaging.

The controller may be configured to detect if the region of interest is present using a model-based reconstruction of the anatomy from the 3D images. The controller is for example configured to use the model-based reconstruction to infer the anatomy outside of the field of view volume by means of a statistical prior.

The statistical prior is a probability distribution allowing the continuation of the anatomy outside the field of view to be inferred. The statistical prior describes the shape variability. By identifying certain landmarks, the shape distribution can be used to find likely positions of other landmarks, conditioned over the locations of the identified landmarks.

The invention also provides an ultrasound imaging method, comprising:
acquiring real-time 3D images of a field of view volume with a 3D ultrasound imaging probe manually positioned by a user;
performing a real-time image analysis of the 3D images to:
   identify anatomical structure and landmarks in the 3D images;
   based on the identified anatomical structure and landmarks, determine if a region of interest of the target anatomical structure is present;
   if, based on the identified anatomical structure and landmarks, it is determined that the region of interest is not present, to infer the location of the region of interest outside the field of view volume from the anatomical structure and landmarks that are detected within the field of view;
   identify the position of at least one reference anatomical plane for the target anatomical structure; and
   determine a relative position between the reference anatomical plane and a position and orientation of the 3D ultrasound imaging probe; and
provide ultrasound guidance information based on the determined relative position.

Providing guidance information for example comprises:
providing advice as to whether or not imaging a desired reference anatomical plane of the region of interest is achievable from the current position and orientation of the ultrasound imaging probe; or
providing recommendations about the nearest achievable acquisitions of a reference anatomical plane of the region of interest from the current position and orientation of the ultrasound probe; or
providing warnings or guidance to indicate required correction of the probe position to enable acquisition of a desired reference anatomical plane.

The method may further comprise, in response to an input command, acquiring simultaneously a set of views of reference anatomical planes.

The method may comprise detecting if the region of interest is present by:
using a landmarks detection algorithm for tracking key anatomical points; or
using a model-based reconstruction of the anatomy from the 3D images.

A model-based reconstruction may be used to infer the anatomy outside of the field of view volume by means of a statistical prior.

The invention also provides a computer program comprising computer program code means which is adapted, when said program is run on a computer, to implement the method defined above.

The invention also provides a computer program comprising computer program code means which is adapted, when said program is run on a computer, to implement the method defined above.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Figure 1 shows an ultrasound imaging system;
Figure 2 shows an image of a heart, which has been segmented by an automatic segmentation algorithm, with an initial view and a view after moving the ultrasound probe in response to guidance; and
Figure 3 shows an ultrasound imaging method.

### DETAILED DESCRIPTION OF EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides an ultrasound imaging system which performs real-time image analysis of the 3D ultrasound images to identify anatomical structure and landmarks in the 3D images, so that it can be determined if a region of interest of a target anatomical structure is present. If the region of interest is not present, the location of the region of interest outside the field of view volume is inferred from the anatomical structure and landmarks that are detected within the field of view. A relative displacement between the reference anatomical plane and a position and orientation of the 3D ultrasound imaging probe can then be derived so that ultrasound guidance information can be given.

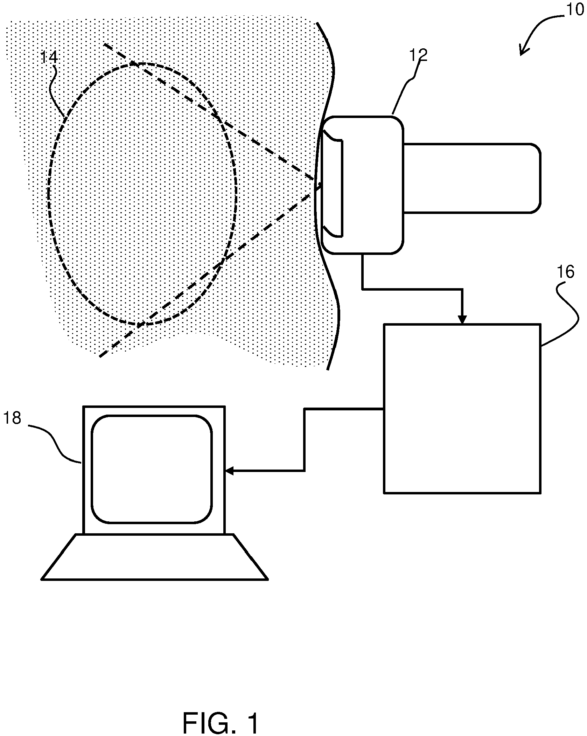

Figure 1 shows an ultrasound imaging system 10, comprising a 3D ultrasound imaging probe 12 for acquiring real-time 3D images of a field of view volume 14. The 3D ultrasound imaging probe 12 is manually positioned by a user.

The 3D Ultrasound imaging probe 12 provides continuous capture of ultrasound volumes and hence operates in a live mode. This continuous stream of ultrasound volumes may be visualized by a sonographer, or it may be acquired in the background. A real-time image quality analysis is optionally performed of the volumes to detect if the images are of sufficient quality for further processing. This analysis may filter out imaged volumes for which a target anatomical structure (e.g. an organ of interest) is not present, or volumes of insufficient image quality.

The image data is provided to a controller 16 which performs real-time image analysis of the 3D images.

This image analysis aims to detect a position and orientation of the imaging probe 12 compared to a position and orientation which would result in a desired reference image, i.e. a standard image from which a standard diagnosis can be made. The controller connects to a display 18 which functions as a user interface and provides ultrasound guidance information, for example to assist the user in repositioning the ultrasound probe to enable the desired image to be taken.

The image analysis involves identifying anatomical structure and landmarks in the 3D images. This may make use of a landmarks detection algorithm (e.g. to detect key anatomical points such as the valves in cardiac imaging) or a fast model-based reconstruction of the anatomy may be performed that can infer a mesh of the anatomy from the input image. Such a mesh can also encode anatomical planes of reference (e.g. 4-Chambers plane in cardiac imaging).

The desired reference image, e.g. from which particular measures of diagnostic relevance may be derived, requires a particular region of interest to be visible in the image. This region of interest may be a particular part of a target anatomical structure such as a part of an organ or vessel, or it may be a complete organ, or an image of a set of anatomic features such as a set of heart valves, or it may be a particular imaging plane through a structure. For example, a region of interest may be a particular heart valve or the 4-chambers plane. The target anatomical structure in this example is then the heart, and the region of interest is the part of the heart which needs to be imaged in order to enable the desired diagnostic evaluation. The region of interest may however in some cases be the whole of an organ.

Based on the identified anatomical structure, it can be determined if the required region of interest (for the particular reference view which is being sought) is present. The invention enables guidance to be provided even when it is determined that the region of interest is not present. For example, in the case of the heart, the left ventricle (LV) long axis may be identified and the apex position may be predicted. This may reveal that the target long axis (or some of it) and the apex are outside of the current field of view.

A model-based reconstruction may for example infer the anatomy outside of the field of view volume by means of a statistical prior. The statistical prior is a probability distribution allowing the continuation of the anatomy outside the field of view to be inferred. By identifying certain landmarks, a shape distribution can be used to find likely positions of other landmarks, conditioned over the locations of the identified landmarks.

The location of the region of interest outside the field of view volume 14 is in this way determined by inferring the location from the anatomical structure and landmarks that are detected within the field of view 14.

Using the inferred location of the region of interest (or the locations of landmarks within the region of interest), the position of the reference anatomical plane can then be derived. By determining a relative displacement (i.e. translational offset and angular offset) between the reference anatomical plane and a position and orientation of the actual current 3D ultrasound imaging probe, the guidance information can be derived. For example, the controller can compute distance measurements between planes and/or landmarks from the identified anatomical structure and the current ultrasound acquisition axes.

The guidance may for example also give recommendations about the reference acquisitions which can be made with the least movement from the current probe position. These acquisitions may be anatomical planes (e.g. 4-Chambers view) or acquisitions directed to specific anatomical structures (e.g. valves).

In a preferred example, the guidance is manual guidance advice to assist in capturing one of the reference anatomical planes i.e. reference views. The guidance then advises of the required corrections to be made in terms of probe motion (translation and/or rotation) to achieve the nearest reference view. The recommendations can be implemented as automated buttons or implemented as voice over commands or graphical output.

The analysis of the anatomical structure makes use of so-called anatomical intelligence, by which 3D anatomical models are used which provide organ modeling, image slicing and quantification. This image analysis can run in the background. In this way, anatomical intelligence is used as part of a continuous background task, rather than being performed only on isolated set of volumes. This can make echocardiography more intuitive by understanding what the sonographer is trying to achieve, giving hints about how to optimize the probe placement, and proposing tuned acquisition modes for standard parts of the echocardiographic exam.

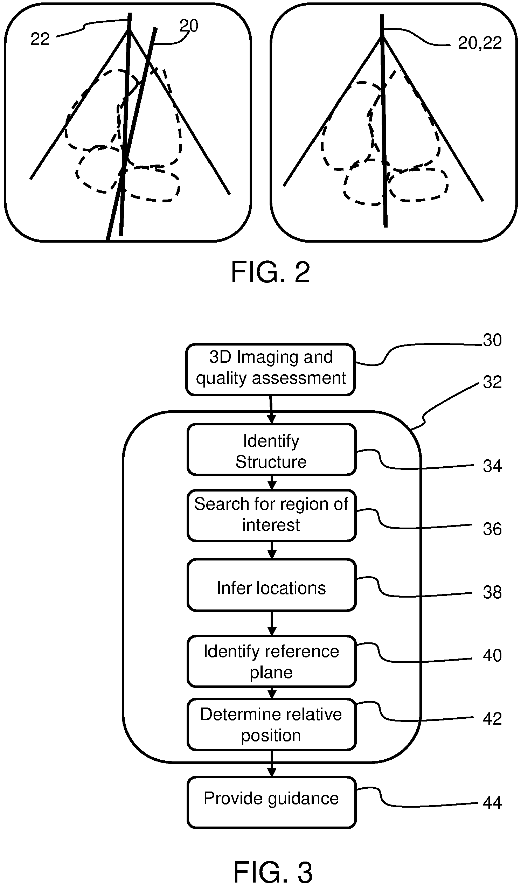

Figure 2 shows an image of a heart, which has been segmented by an automatic segmentation algorithm. The left image shows an initial view captured by the ultrasound probe.

The quality assessment verifies the presence of the heart in the field of view and by identifying anatomical structure and landmarks in the 3D images, cardiac features such as cardiac valves are detected and localized. For example for cardiac apical view analysis, the left ventricular apex, the mitral valve and the tricuspid valve may be identified.

The recommendation of which reference images to take for example does not appear when the heart (or more generally the organ of interest) is not sufficiently within the ultrasound field of view, or when the image quality is too low for deriving any recommendation.

In one example, the controller computes a distance and/or angular orientation offset between the LV long axis (labeled 20 in Figure 2) and the ultrasound acquisition axis (labeled 22 in Figure 2).

The recommendation system may alert the sonographer when the LV long axis is too distant from the ultrasound acquisition axis, thus impeding the acquisition of a standard apical view (2, 3 or 4-Chambers View). Thus, if the distance and/or angular orientation exceeds a certain threshold, a warning message is displayed.

The recommendation system may then calculate and indicate the ultrasonic probe motion necessary to correct the current probe position and achieve the nearest reference view. The required corrective probe motion is schematically displayed to guide the sonographer, for example as displayed probe movement instructions.

The probe is then moved to the position shown in the right image in Figure 2 with the axes 20,22 aligned. When the ultrasound and LV long axes align well, a confirmation is for example displayed (either graphically or with a message).

The user interface may include output buttons for the selection of standard 2D planar acquisitions (possibly batched) such as the 2-, 3- and 4-Chambers views. These buttons allow to automatically switch to one or several planes of reference.

Another example of the possible use of the system is for the analysis of the cardiac parasternal view. The controller then identifies the valve and the 3D geometry of the heart chambers such as the aortic root and the left atrium. The recommendation system in this case alerts the sonographer when the acquisition plane cuts the aortic root and the left atrium at their maximal extent. The guidance system may in this case indicate the amount and direction of corrective tilting to reach the optimal long axis parasternal view.

Another example of the use of the system is for zooming in on a valve and visualizing it in 3D. The 3D volume can contain one or multiple layers showing the B-Mode and the color Doppler images.

The sonographer can also use the system to switch to a Doppler acquisition. For this purpose, a Doppler gate may be automatically placed at a certain distance of the valve. The Doppler line is also set automatically, in a direction normal to the valve plane.

Figure 3 shows a computer-implemented ultrasound imaging method.

In step 30, real-time 3D images of a field of view volume are acquired with a 3D ultrasound imaging probe, manually positioned by a user. This step may include image quality assessment to determine if the 3D volume image contains the anatomical region of interest and has sufficient image quality. This may involve use of a trained classifier which learns if the anatomy detection is reliable from the image of the type that has been received.

In the set of steps 32, real-time image analysis of the 3D images is performed.

In step 34 anatomical structure and landmarks are identified in the 3D images. In step 36, based on the identified anatomical structure, it is determined if a region of interest of the target anatomical structure is present. If it is present, no inference of location outside the field of view is needed.

However, if based on the identified anatomical structure, it is determined that the region of interest is not (fully) present, the location of the region of interest outside the field of view volume is inferred in step 38 from the anatomical structure and landmarks that are detected within the field of view.

In step 40, the position of at least one reference anatomical plane for the target anatomical structure is identified.

A relative position (in terms of translation and rotation) between the reference anatomical plane and a position and orientation of the 3D ultrasound imaging probe can then be derived in step 42.

Ultrasound guidance information is then provided in step 44 based on the determined relative position. This may be advice as to whether or not imaging a desired reference anatomical plane of the region of interest is achievable from the current position and orientation of the ultrasound imaging probe and/or it may be recommendations about the nearest achievable acquisitions of a reference anatomical plane of the region of interest from the current position and orientation of the ultrasound probe and/or providing warnings or guidance to indicate required correction of the probe position to enable acquisition of a desired reference anatomical plane.

The use of anatomical intelligence in the image analysis system enables a number of workflow improvements. Shortcuts may for example be provided to one or several (consecutive) reference views, that are identified as feasible from the current probe position, and for which the plane or the focus area have been computed by anatomical intelligence. These shortcuts may be displayed to the user.

Preset acquisitions may for example be proposed centered on anatomical landmarks, such as the valves. An input command may be provided to acquire simultaneously a set of views of reference anatomical planes.

The processing of ultrasound echo signals to create the ultrasound images has not been described above, as this is entirely conventional. Similarly, the segmentation of ultrasound images to identify landmarks as well as anatomical structures is entirely conventional.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to".

Any reference signs in the claims should not be construed as limiting the scope.

## Claims

1. An ultrasound imaging system (10), comprising:
a 3D ultrasound imaging probe (12) for acquiring real-time 3D images of a field of view volume with the 3D ultrasound imaging probe manually positioned by a user;
a controller (16) configured to:
perform a real-time image analysis of the 3D images thereby to:
identify anatomical structure and landmarks in the 3D images;
based on the identified anatomical structure and landmarks, determine if a region of interest of a target anatomical structure is present;
if, based on the identified anatomical structure and landmarks, it is determined that the region of interest is not present, to infer the location of the region of interest outside the field of view volume from the anatomical structure and landmarks that are detected within the field of view;
identify the position of at least one reference anatomical plane for the target anatomical structure;
determine a relative position between the reference anatomical plane and a position and orientation of the 3D ultrasound imaging probe; and
provide ultrasound guidance information based on the determined relative position.

2. The system of claim 1, wherein the controller is further configured to perform an image quality assessment for the field of view volume and discard images determined as unsuitable for the identification of anatomical structure and landmarks.

3. The system of claim 1 or 2, wherein the controller is configured to determine the relative position by:
identifying one or more main directions of the anatomy of the target of interest; and
determining a distance and orientation between the one or main directions and one or more axes of the ultrasound imaging probe.

4. The system of any one of claims 1 to 3, wherein the guidance information provides:
advice as to whether or not imaging a desired reference anatomical plane of the region of interest is achievable from the current position and orientation of the ultrasound imaging probe; or
recommendations about the nearest achievable acquisitions of a reference anatomical plane of the region of interest from the current position and orientation of the ultrasound probe; or
warnings or guidance to indicate required correction of the probe position to enable acquisition of a desired reference anatomical plane.

5. The system of any one of claims 1 to 4, wherein the controller is configured, in response to an input command, to acquire simultaneously a set of views of reference anatomical planes.

6. The system of any one of claim 1 to 5, wherein the controller is configured to detect if the region of interest is present by using a landmarks detection algorithm for tracking key anatomical points.

7. The system of claim 6, wherein the key anatomical points comprise the heart valves for cardiac imaging.

8. The system of any one of claims 1 to 7, wherein the controller is configured to detect if the region of interest is present using a model-based reconstruction of the anatomy from the 3D images.

9. The system of claim 8, wherein the controller is configured to use the model-based reconstruction to infer the anatomy outside of the field of view volume by means of a statistical prior.

10. An ultrasound imaging method, comprising:
(30) acquiring real-time 3D images of a field of view volume with a 3D ultrasound imaging probe manually positioned by a user;
(32) performing a real-time image analysis of the 3D images to:
(34) identify anatomical structure and landmarks in the 3D images;
(36) based on the identified anatomical structure and landmarks, determine if a region of interest of the target anatomical structure is present;
(38) if, based on the identified anatomical structure and landmarks, it is determined that the region of interest is not present, to infer the location of the region of interest outside the field of view volume from the anatomical structure and landmarks that are detected within the field of view;
(40) identify the position of at least one reference anatomical plane for the target anatomical structure; and
(42) determine a relative position between the reference anatomical plane and a position and orientation of the 3D ultrasound imaging probe; and
(44) provide ultrasound guidance information based on the determined relative position.

11. The method of claim 10, wherein providing guidance information comprises:
providing advice as to whether or not imaging a desired reference anatomical plane of the region of interest is achievable from the current position and orientation of the ultrasound imaging probe; or
providing recommendations about the nearest achievable acquisitions of a reference anatomical plane of the region of interest from the current position and orientation of the ultrasound probe; or
providing warnings or guidance to indicate required correction of the probe position to enable acquisition of a desired reference anatomical plane.

12. The method of claim 10 or 11, comprising, in response to an input command, acquiring simultaneously a set of views of reference anatomical planes.

13. The method of any one of claims 10 to 12, comprising detecting if the region of interest is present by:
using a landmarks detection algorithm for tracking key anatomical points; or
using a model-based reconstruction of the anatomy from the 3D images.

14. The method of claim 13, comprising using a model-based reconstruction to infer the anatomy outside of the field of view volume by means of a statistical prior.

15. A computer program comprising computer program code means which is adapted, when said program is run on a computer, to implement the method of any one of claims 10 to 14.
